# EUROPÄISCHE PATENTSCHRIFT

(11) **EP 0 654 469 B1**
(45) Veröffentlichungstag und Bekanntmachung des Hinweises auf die Patenterteilung: **22.08.2001**
(21) Anmeldenummer: 94810651.3
(22) Anmeldetag: 14.11.1994
(51) Int. Cl.: C07D 251/24, C08K 5/3492

(54) **o-Hydroxyphenyl-s-triazine enthaltende stabilisierte Polymere**
Stabilised polymers containing o-hydroxyphenyl-s-triazines
Polymères stabilisés contenant des o-hydroxyphényl-s-triazines

(30) Priorität: 23.11.1993 CH 348893
(43) Veröffentlichungstag der Anmeldung: 24.05.1995
(73) Patentinhaber: Ciba Specialty Chemicals Holding Inc., 4057 Basel (CH)
(72) Erfinder: Birbaum, Jean-Luc, Dr., CH-1700 Fribourg 4 (CH); Kaschig, Jürgen, Dr., D-79104 Freiburg (DE); Reinehr, Dieter, Dr., D-79400 Kandern (DE); Rembold, Manfred, Dr., CH-4148 Pfeffingen (CH); Schmitter, André, Dr., F-68220 Hegenheim (FR); Luther, Helmut, Dr., D-79639 Grenzach-Wyhlen (DE); Herzog, Bernd, Dr., D-79713 Bad Säckingen (DE); Hüglin, Dietmar, Dr., D-79104 Freiburg (DE)

(56) Entgegenhaltungen:
- EP-A- 0 444 323
- EP-A- 0 649 841
- CH-A- 480 090
- DE-A- 1 469 811

## Beschreibung

Die vorliegende Erfindung betrifft organische Polymere enthaltend als Stabilisatoren Verbindungen vom Typ o-Hydroxyphenyl-s-triazin, die mindestens zwei Alkoxyphenyl-Substituenten enthalten, die Verwendung dieser Verbindungen als Stabilisatoren für organische Polymere sowie das mit diesen Verbindungen stabilisierte Polymer.

Einzelne Verbindungen vom Typ o-Hydroxyphenyl-s-triazin, darunter 2-(2-Hydroxyphenyl)-4,6-bis(4-methoxyphenyl)-1,3,5-triazin, werden in DE-A-1469811 als Stabilisator für organische Polymere vorgeschlagen.

EP-A-0 649 841 (ein Dokument gemäss Artikel 54(3) EPÜ) offenbart ein Verfahren zur Herstellung von hydroxyphenylsubstituierten 1,3,5-Triazinen.

Die neuen stabilisierten organischen Polymere enthalten 0,01 bis 5 Gew.-%, bezogen auf das organische Polymer, mindestens eines o-Hydroxyphenyl-s-triazins der Formel (2) 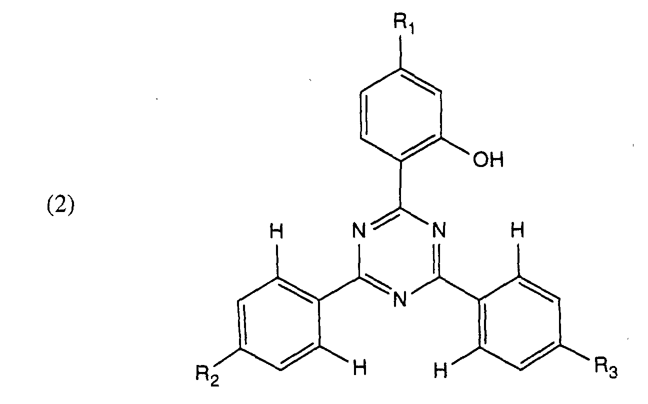 worin
R₁ Hydroxy, C₁-C₁₅-Alkyl, C₁-C₁₅-Alkoxy oder einen Rest der Formel 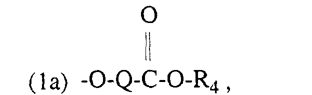
R₂ und R₃, unabhängig voneinander, Wasserstoff oder C₁-C₁₅-Alkoxy,
R₄ C₁-C₅-Alkyl oder C₁-C₅-Alkoxy-C₁-C₅-Alkyl und
Q einen C₁-C₄-Alkylenrest bedeuten, und
die Verbindung mindestens zwei C₁-C₁₅-Alkoxyreste aufweisen muss.

C₁-C₁₅-Alkyl und C₁-C₁₅-Alkoxy sind geradkettige oder verzweigte Alkyl- bzw. Alkoxyreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl oder Pantadecyl, bzw. Methoxy, Ethoxy, Propoxy, Butoxy, Pentyloxy, Hexyloxy, Heptyloxy, Octyloxy, Isooctyloxy, Nonyloxy, Undecyloxy, Dodecyloxy, Tetradecyloxy oder Pentadecyloxy.

Der zweiwertige Rest Q enthält vorzugsweise 2 bis 4 Kohlenstoffatome. Bevorzugte zweiwertige Alkylenreste sind Ethylen-, Ethylenpropylen- oder Ethylenisopropylen-Reste.

Vorzugsweise kommen solche stabilisierten Polymere in Betracht, worin in Verbindungen der Formel (2)
R₁ Hydroxy, C₁-C₁₅-Alkyl, C₁-C₁₅-Alkoxy und
R₂ und R₃ unabhängig voneinander Wasserstoff oder C₁-C₁₅-Alkoxy bedeuten.

Im Vordergrund des Interesses stehen Verbindungen der Formel (2),
worin R₂ und R₃, unabhängig voneinander, C₁-C₅-Alkoxy bedeuten.

Bevorzugt sind dabei solche Triazinverbindungen, bei denen R₂ Wasserstoff bedeutet, oder bei denen
R₂ und R₃ Methoxy oder Ethoxy sind, und insbesondere solche, bei denen
R₂ Wasserstoff und
R₃ Methoxy oder Ethoxy, oder solche, bei denen
R₁ und R₃ Methoxy und
R₂ Wasserstoff bedeuten.

Weiterhin sind Hydroxyphenyl-s-triazine der Formel (2) bevorzugt, worin
R₁, R₂ und R₃, unabhängig voneinander, C₅-C₁₅-Alkoxy, oder solche Verbindungen, bei denen
R₁ einen Rest der Formel (1a) und
R₂ und R₃ C₅-C₁₅-Alkoxy bedeuten.

Als Beispiele für Verbindungen der Formel (2) seien genannt:
2-(2',4'-Dihydroxyphenyl)-4,6-bis(4-methoxyphenyl)-1,3,5-triazin
2-(2'-Hydroxy-4-methoxyphenyl)-4,6-bis(4-methoxyphenyl)-1,3,5-triazin
2-(2'-Hydroxy-4-Hexyloxyphenyl)-4,6-bis(4-methoxyphenyl)-1,3,5-triazin
2-(2-Hydroxy-4-methoxyphenyl)-4-(4-methoxyphenyl)-6-phenyl-1,3,5-triazin
2-(2'-Hydroxy-4'-[2-ethylhexyloxy])-4,6-bis-(2-ethylhexyloxy)-phenyl-1,3,5-triazin

Die Verbindungen der Formel (2) lassen sich nach verschiedenen Verfahren herstellen.

Beispielsweise lassen sich die Verbindungen der Formel (2) in einem einstufigen Verfahren durch Umsetzung einer Salicylverbindung mit einer Benzamidinverbindung herstellen.

Bei den Ausgangsverbindungen handelt es sich um substituierte Salicylsäureester oder Salicylsäurehalogenide, wie z.B. Salicylsäuremethyl-, Salicylsäureethyl- oder Salicylsäurepropylester, bzw. Salicylsäurechlorid oder -bromid, die im Phenylrest, entsprechend der Bedeutung von R₁ substituiert sind.

Ist die Ausgangsverbindung ein Salicylsäurehalogenid, beträgt das molare Verhältnis der Salicylverbindung zur Benzamidinverbindung vorzugsweise 1:3 bis 1:2.

Wird als Ausgangsverbindung ein Salicylsäureester eingesetzt, beträgt das molare Verhältnis der Salicylverbindung zur Benzamidinverbindung vorzugsweise 2:1 bis 1:2.

Als Benzamidinverbindungen kommen das Benzamidinhydrobromid und vorzugsweise das -chlorid, die im Phenylrest entsprechend der Bedeutung von B weiter substituiert sein können, in Betracht. Diese Verbindungen werden normalerweise als Festprodukte mit einem Gehalt an Aktivsubstanz von ca. 90-95% eingesetzt.

Verwendet man als Salicylverbindung ein Salicylsäurehalogenid, wird in der Regel zur Neutralisation der bei der Reaktion entstehenden Säure mindestens die berechnete Menge einer Base zugegeben. Als Basen können sowohl organische als auch anorganische Verbindungen verwendet werden, wie z.B. Alkalihydroxid, insbesondere Natron- oder Kalilauge; wässrige Ammoniaklösung; Ammoniakgas; Alkalicarbonat, insbesondere Natrium- oder Kaliumcarbonat; Natriumacetat; tert. Amine wie Pyridin oder Trialkylamine, insbesondere Triethylamin, N,N-Dimethylcyclohexylamin, N,N-Dimethylanilin; Alkalialkylate, insbesodere Natrium- und Kaliummethylat oder Kalium-tert.butylat.

Das Verfahren wird in der Regel so durchgeführt, dass man die Salicyl- und die Benzamidinverbindung in einem inerten Lösungsmittel vorlegt.

Als inerte Lösungsmittel kommen dabei aliphatische Kohlenwasserstoffe und deren Mischungen, wie z.B. Cyclohexan oder aromatische Kohlenwasserstoffe wie Toluol, oder Dimethylacetamid oder Mischungen dieser Lösungsmittel in Betracht.

Verwendet man ein Salicylsäurehalogenid, kann noch ein weiteres, in der Regel polares Lösungsmittel, wie z.B. Acetonitril oder Dioxan zugesetzt werden.

Die Reaktionszeit beträgt im allgemeinen 2 bis 30 Stunden. Je nachdem, ob man als Ausgangsverbindung ein Salicylsäurehalogenid oder einen Salicylsäureester einsetzt, können die Reaktionszeiten variieren. Verwendet man einen Salicylsäureester, beträgt die Reaktionszeit vorzugsweise 4 bis 30 Stunden, insbesondere 18 bis 22 Stunden. Verwendet man ein Salicylsäurehalogenid, sind die Reaktionszeiten etwas kürzer. Sie betragen vorzugsweise 2 bis 20 Stunden, insbesondere 4 bis 8 Stunden.

Die Reaktionen verlaufen in der Regel leicht exotherm ab. Allerdings sollte eine Reaktionstemperatur von 95°C nicht überschritten werden, da bei höheren Temperaturen Nebenprodukte entstehen können, wie beispielsweise Nitrilverbindungen aus den Benzamidinen. In der Praxis wird die Reaktion in einem Temperaturbereich von 60 bis 95°C, vorzugsweise 80 bis 95°C durchgeführt.

Weiterhin können die erfindungsgemässen Verbindungen der Formel (1) durch Dehydrierung einer Dihydrotriazinverbindung der Formel 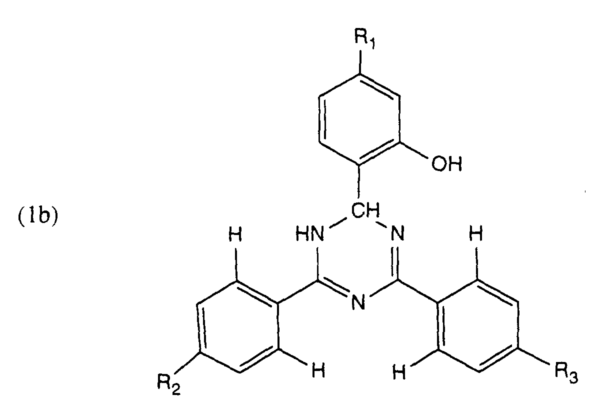 hergestellt werden. R₁, R₂, R₃ haben dabei die in Formel (2) angegebene Bedeutung.

Als Dehydrierungsmittel wird in der Regel Chloranil eingesetzt. Die Dehydrierung von Dihydrotriazinverbindungen zu 1,3,5-Triazinen mit Hilfe von Chloranil ist z.B. aus der Khim. Geteritsikl. Soedin. (2), S. 350-353 (1969) bekannt.

Die Herstellung der Ausgangsverbindungen der Formel (1b) erfolgt in an sich bekannter Weise durch Umsetzung von 2 Mol einer entsprechenden Benzamidinhydrohalogenidverbindung mit einem Mol einer entsprechenden α-Hydroxybenzaldehydverbindung.

Ein weiterer Weg zur Herstellung der Triazinverbindungen der Formel (2) besteht in der Umsetzung einer Monochlortriazinverbindung der Formel 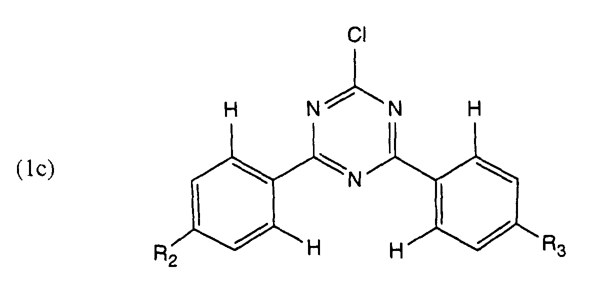 mit einer α-Hydoxyphenylverbindung der Formel 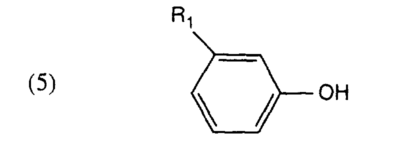 in Gegenwart einerLewis-Säure, insbesondere Aluminiumchlorid.

R₁, R₂, R₃ haben dabei die in Formel (2) angegebene Bedeutung. Diese Reaktion ist zum Beispiel aus der J. Am. Chem. Soc. 73(7) (1951) bekannt.

Die Ausgangsverbindungen der Formel (lc) lassen sich in an sich bekannter Weise z.B. durch Umsetzung von Cyanurchlorid und den entsprechenden Phenylmagnesiumbromidverbindungen in einer Grignardreaktion herstellen. Diese Reaktion ist z.B. durch Hirt et al., Helv. Chim. Acta, 33, 1368 (1950) bekannt.

Die Verbindungen der Formel (2) eignen sich als UV-Stabilisatoren, d.h. zum Schützen von ultraviolett empfindlichen organischen Materialien vor der schädigenden Einwirkung von Ultraviolettstrahlung.

Die Verbindungen der Formel (2) werden vorteilhaft als Stabilisatoren für organische Polymere gegen deren Schädigung durch Licht, Sauerstoff und Wärme verwendet. Die Erfindung betrifft daher auch ein Verfahren zum Stabilisieren organischer Polymere gegen Schädigung durch Licht, Sauerstoff und Wärme, das dadurch gekennzeichnet ist, daß man diesen Materialien mindestens eine Verbindung der Formel (2) in der oben genannten Menge beimischt. Beispiele für solche zu stabilisierenden Polymere sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beispielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polpropylenoxid oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyhamstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Besonders bevorzugt ist die Verwendung der erfindungsgemäßen Verbindungen als Stabilisatoren in Lacken aller Art. Dies bedeutet auch ein Verfahren gemäß obiger Beschreibung, worin das organische Polymer ein Bindemittel für einen Lack ist. Bei den Lacken kann es sich um pigmentierte oder unpigmentierte Lacke oder Metalleffektlacke handeln. Sie können ein organisches Lösungsmittel enthalten oder lösungsmittelfrei sein oder wässrige Lacke sein.

Die Lacke können als Bindemittel mindestens eines der vorhin aufgeführten Polymeren enthalten. Beispiele für Lacke mit speziellen Bindemitteln sind die folgenden:
1. Lacke auf Basis von kalt- oder heiß-vernetzbaren Alkyd-, Acrylat-, Polyester-, Epoxid- oder Melaminharzen oder Mischungen solcher Harze, gegebenenfalls mit Zusatz eines sauren Härtungskatalysators;
2. Zweikomponenten-Polyurethanlacke auf Basis von hydroxylgruppenhaltigen Acrylat-, Polyester- oder Polyetherharzen und aliphatischen oder aroma- tischen Polyisocyanaten;
3. Einkomponenten-Polyurethanlacke auf Basis von blockierten Polyisocyanaten, die während des Einbrennens deblockiert werden;
4. Zweikomponentenlacke auf Basis von (Poly)ketiminen und aliphatischen oder aromatischen Polyisocyanaten;
5. Zweikomponentenlacke auf Basis von (Poly)ketiminen und einem ungesättigten Acrylatharz oder einem Polyacetoacetatharz oder einem Methylacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten.
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis siloxanmodifizierter oder fluormodifizierter Acrylatharze.

Die Lacke können auch strahlenhärtbare Lacke sein. In diesem Fall besteht das Bindemittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemäßen Stabilisatoren vorzugsweise der unpigmentierten obersten Schicht zugesetzt werden.

Die Lacke können auf die Substrate (Metall, Plastik, Holz etc.) nach den üblichen Verfahren aufgebracht werden, beispielsweise durch Streichen, Besprühen, Gießen, Tauchen oder Elektrophorese.

Die Menge des zugesetzten Stabilisators der Formel (2) hängt vom jeweiligen Substrat und dessen Verwendungszweck ab. Bevorzugt verwendet man 0,05 bis 3 Gew.-%, bezogen auf das zu stabilisierende Polymer. Erfindungsgemäß sind daher insbesondere Polymere enthaltend 0,01 bis 5 Gew.-%, vor allem 0,05 bis 3 Gew.-%, mindestens einer Verbindung der Formel (2) geeignet.

In bestimmten Fällen kann es von Vorteil sein, zwei oder mehrere der Verbindungen der Formel (2) zu verwenden. Außerdem können ein oder mehrere andere Stabilisatoren und/oder sonstigen Zusatzstoffe mitverwendet werden, wie z.B. die folgenden Typen von Verbindungen:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecyl-thiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methyl-cyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.
   1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.12. Ester der β-(3,5-Di-tert-butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.13. Ester der ß-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Amide der β-(3,5-Di-tert-butyl-4-hydroxyphenyl-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel
   2.1. 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol, 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'-hydroxyphenyl)-5-chlor-benztriazol, 2-(3'-tert.Butyl- 2'-hydroxy-5'-methylphenyl)-5-chlor-benztriazol, 2-(3'-sec-Butyl-5'-tert-butyl-2'-hydroxyphenyl)-benztriazol, 2-(2'-Hydroxy-4'-octoxyphenyl)-benztriazol, 2-(3',5'-Di-tert-amyl-2'-hydroxyphenyl)-benztriazol, 2-(3',5'-Bis-(α,α-dimethylbenzyl)-2'-hydroxyphenyl)-benztriazol, Mischung aus 2-(3'-tert-Butyl-2'-hydroxy-5'-(2-octyloxycarbonylethyl)-phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'-hydroxy-5'-(2-methoxycarbonylethyl)phenyl)-5-chlor-benztriazol, 2-(3'-tert-Butyl-2'-hydroxy-5'-(2-methoxycarbonylethyl)phenyl)-benztriazol, 2-(3'-tert-Butyl-2'-hydroxy-5'-(2-octyloxycarbonylethyl)-phenyl)-benztriazol, 2-(3'-tert-Butyl-5'-[2-(2-ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; [R-CH₂CH₂-COO(CH₂)₃ 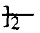 mit R = 3'-tert-Butyl-4'-hydroxy-5'-2H-benztriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbo-methoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxy-phenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Weitere Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863 oder US-A-4 338 244 beschrieben.

Von besonderer Bedeutung sind stabilisierte Polymere, welche einen zusätzlichen Gehalt an einem Lichtschutzmittel aus der Klasse der sterisch gehinderten Amine oder/und aus der Klasse der 2-(2'-Hydroxyphenyl)-benztriazole aufweisen. Unter den sterisch gehinderten Aminen sind insbesondere solche Verbindungen zu verstehen, die im Molekül eine oder mehrere Gruppen der Formel 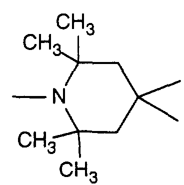 enthalten, wobei diese

Verbindungen monomer, oligomer oder polymer sein können. Beispiele für solche Verbindungen sind obigem Punkt 2.6. der Liste von möglichen zusätzlichen Stabilisatoren zu entnehmen.

Der Zusatz der Verbindungen der Formel (2) sowie gegebenenfalls weiterer Zusatzstoffe zu den Polymeren kann vor oder während der formgebenden Verarbeitung der Polymeren erfolgen, beispielsweise durch Mischen in Pulverform oder durch Zusatz zur Schmelze oder Lösung des Polymeren bzw. zu einer geeigneten Lackformulierung, die einen polymeren Binder enthält.

Die Erfindung betrifft daher auch die durch Zusatz mindestens einer Verbindung der Formel (2) stabilisierten Polymeren, die gegebenenfalls noch andere Zusatzstoffe enthalten können. Die so stabilisierten Polymeren können in verschiedener Form verwendet werden, wie z.B. als Fasern, Folien, Faserbändchen, Profile, Hohlkörper, Platten, Doppelstegplatten oder als Bindemittel für Lacke, Anstriche, Klebstoffe und Kitte. Von besonderem Interesse ist ihre Verwendung in Lacken.

In den folgenden Beispielen beziehen sich die Prozentsätze auf das Gewicht. Die Mengen beziehen sich bei den Farbstoffen und bei den Triazinverbindungen auf die Reinsubstanz.

### Vergleichsbeispiel 1: 2-(2-Hydroxyphenyl)-4,6-bis(4-methoxyphenyl)-1,3,5-triazin (Beispiel 14 der DE-A-1469811)

42 g 4-Methoxy-benzamidinhydrochlorid werden in 100 ml Dimethylacetamid vorgelegt. Unter Rühren werden 41,2 ml einer 30%igen Natriummethylatlösung zugegeben. Nach weiterer Zugabe von 33 g Salicylsäuremethylester wird auf 90 bis 95°C erwärmt und während 20 Stunden bei dieser Temperatur gerührt. In den ersten drei Stunden destillieren ca. 62 ml eines Gemisches aus Methanol, Dimethylacetamid und Wasser ab. Es wird mit 150 ml Methanol versetzt, auf 5°C abgekühlt und abfiltriert. Nach dem Trocknen bei 110°C erhält man 30,6 g eines hellgelben Produktes der Formel 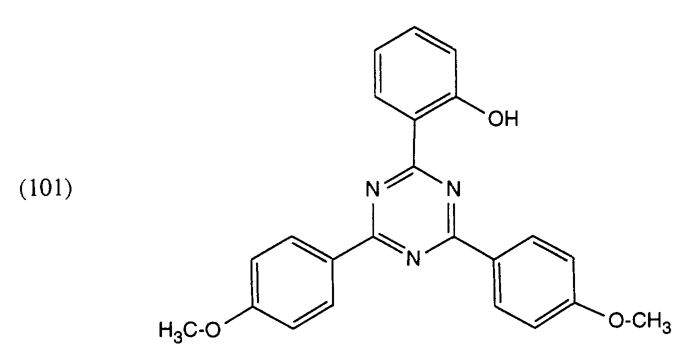 Fp. 205-206°C

Beispiele 2 und 4: Die Verbindungen entsprechend den Formeln (102) und (104) in Tabelle 1 werden analog hergestellt.

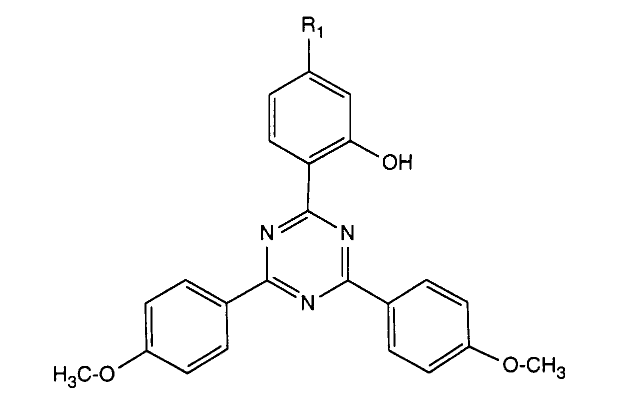

**Tabelle 1:**

| Beispiel | Verbindung | R₁ | R₂ | R₃ | Fₚ [°C] |
|---|---|---|---|---|---|
| 2 | (102) | H | OH | H | 251-252 |
| 4 | (104) | H | CH₃ | H | 211-212 |

### Beispiel 6a (Vergleich):

37,2 g 4-Methoxy-benzamidinhydrochlorid werden zusammen mit 100 ml Methanol vorgelegt. Es werden dann 36 g einer 30%igen Natriummethylatlösung in Methanol und 15,2 g o-Vanillin zugegeben. Die Mischung wird während 20 Stunden bei 50°C gerührt, dann abgekühlt und mit 100 ml Wasser versetzt. Nach dem Waschen mit einem 1:1-Gemisch aus Methanol/Wasser und Trocknen bei 100°C erhält man 34 g eines hell-beigen Produktes der Formel

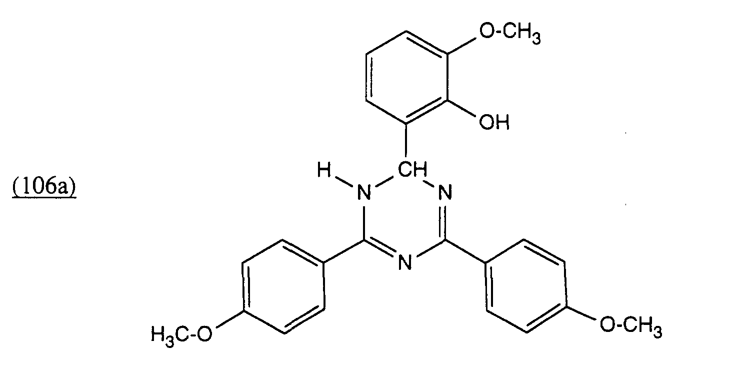

Beispiel 6b: 32 g des Dihydroproduktes (106a) werden in 600 ml Aceton zusammen mit 18,9 g Chloranil vorgelegt und während 20 Stunden bei Raumtemperatur gerührt. Nach dem Abfiltrieren und Trocknen erhält man 27,5 g eines hellen Produktes der Formel 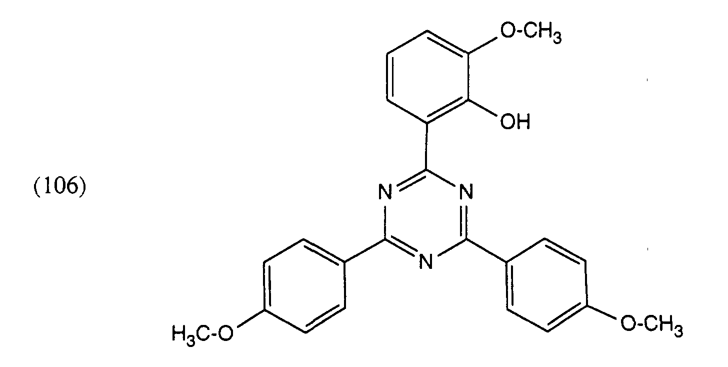 Ausbeute: 86,4% d.Th.
Fp.: 197-198°C

### Beispiel 7: 2-(2-Hydroxy-4-methoxyphenyl)-4,6-bis(4-methoxyphenyl)-1,3,5 -triazin

Man verfährt wie in Beispiel 6 beschrieben, mit dem Unterschied, dass man anstelle von o-Vanillin 2-Hydroxy-4-methoxybenzaldehyd verwendet. Das entprechende Dihydroprodukt der Formel 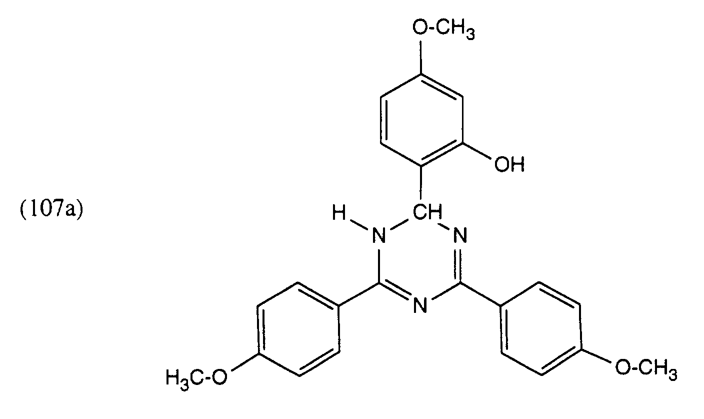 entsteht dabei in einer Ausbeute von 41% d.Th. Die Oxidation mit Chloranil ergibt die Verbindung der Formel 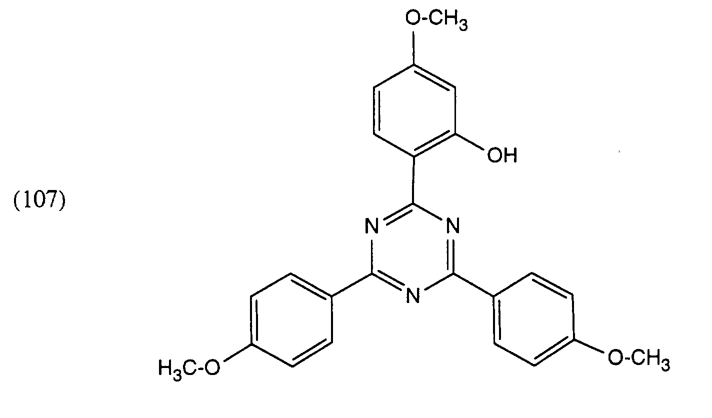 Ausbeute: 77% d.Th.
Fp.: 213-214°C

### Beispiel 8a: 2-Chlor-4,6-bis(4-methoxyphenyl)-1,3,5-triazin

Eine Grignard-Lösung von p-Methoxyphenylmagnesiumbromid (hergestellt aus 12,2 g (0,05 Mol) Magnesium und 93,5 g (0,5 Mol) p-Bromanisol in 130 ml wasserfreiem THF) wird innerhalb von 1,5 Stunden zu einer Lösung von 31,3 g (0,17 Mol) Cyanurchlorid in 100 ml THF gegeben, wobei die Temperatur im Bereich von 0 bis 20°C gehalten wird. Nach beendeter Zugabe wird die Mischung 1,5 Stunden bei Raumtemperatur gerührt, dann auf 150 ml 12%ige Chlorwasserstoff-Lösung im Eisbad gegossen. Die beige Suspension wird abfiltriert, mit Wasser neutral, dann mit Methanol gewaschen. Das Rohprodukt wird aus 350 ml Toluol umkristallisiert. Es entsteht die Verbindung der Formel 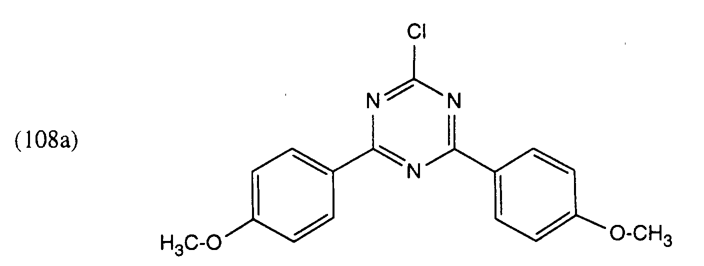 Ausbeute: 40g (72% d.Th.)
Fp.: 193-195°C

### Beispiel 8b: 2-(2,4-Dihydroxyphenyl)-4,6-bis(4-methoxyphenyl)-1,3,5-triazin

Zu einer Mischung aus 23,0 g (0,07 Mol) 2-Chlor-4,6-bis(4-methoxyphenyl)-1,3,5-triazin entsprechend der Formel (108a) und 8,5 g (0,077 Mol) Resorcin in 150 ml Toluol werden 10,3 g (0,077 Mol) Aluminiumchlorid bei 5°C gegeben. Man lässt die Temperatur auf 20°C steigen, dann wird die Mischung 6 Stunden bei 50°C und 24 Stunden unter Rückfluss erhitzt. Die abgekühlte Mischung wird auf 150 ml 12%ige Chlorwasserstofflösung gegossen, das Rohprodukt wird filtriert, mit Wasser neutral, dann mit Methanol gewaschen und getrocknet. Man erhält ein dunkelgelbes Produkt (Vb. 102) der Formel 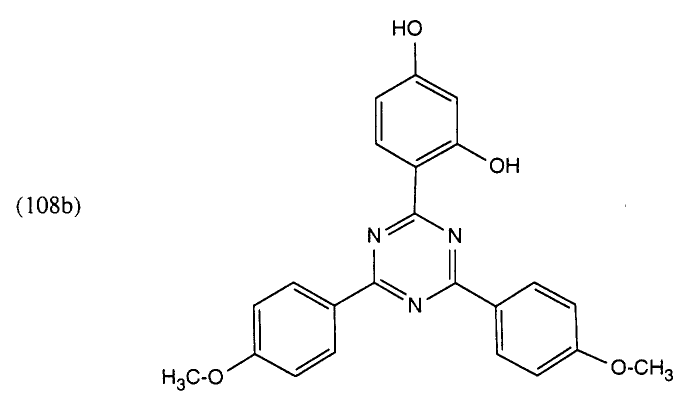 Ausbeute: 18,8 g (67% d.Th.)

### Beispiel 9: 2-Hydroxy-4-Hexyloxyphenyl)-4,6-bis(4-methoxyphenyl)-1,3,5-triazin

15,2 g (0,038 Mol) 2-(2,4-Dihydroxyphenyl)-4,6-bis(4-methoxyphenyl)-1,3,5-triazin entsprechend der Formel (108b), 5,2 g Kaliumcarbonat und 50 mg Kaliumiodid werden 45 Minuten in 100 ml 2-Ethoxyethanol auf 110°C erhitzt, dann werden 6,9 g (0,042 Mol) 1-Bromhexan innerhalb von 15 Minuten zugetropft. Die Mischung wird 12 Stunden bei 110°C gerührt, bei 0°C abgekühlt und filtriert. Das feste Material wird mit Wasser neutral, dann mit Methanol gewaschen und getrocknet. Nach Umkristallisieren aus 2-Ethoxyethanol erhält man das rein hellgelbe Produkt der Formel 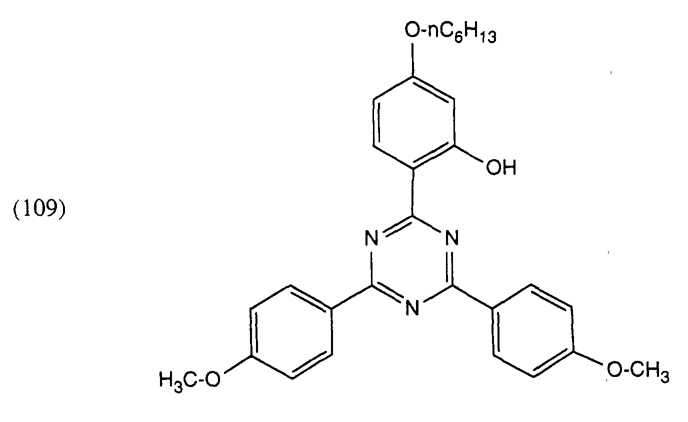 Ausbeute: 4,8 g (26% d. Th.)
Fp.: 123-125°C

### Beispiel 11:

Zu einer Suspension von 2,83 g (10 mmol) 7-Methoxy-2-(4-methoxyphenyl)-4H-1,3-benzoxazin-4-on in 48 ml Methanol werden 4,9 g (10,86 mmol) Benzamidinhydrochlorid (38%ig in Methanol) und anschliessend eine Lösung von 1,95 g (10,86 mmol) Natriummethylat (30%ig in Methanol) zugegeben. Das Gemisch wird zum Sieden erhitzt und mit 38 ml Methanol verdünnt. Nach Kochen unter Rückfluss (30 min) wird der Niederschlag heiss abgesaugt und zweimal mit je 10 ml Methanol gewaschen. Nach Umkristallisieren aus Chloroform/Petrolether werden 3,74 g (97% d. Theorie) des Produktes der Formel 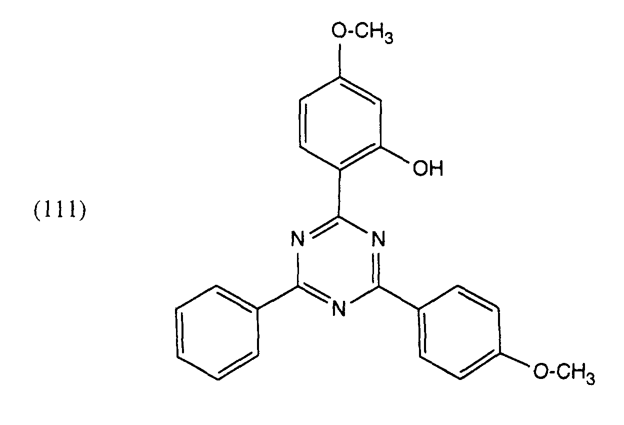 erhalten.
Fp.: 171-172°C
UV-Spektrum (2,25·10⁻⁵ mol in Chloroform)
- λₘₐₓ/εₘₐₓ =: 297/34920
325/Schulter

### Beispiel 12: 2-{4,6-Bis-[4-(2-ethylhexyloxy)-phenyl]-s-triazin-2-yl}-5-(2-ethylhexyloxy)-phenol

a) In einem 11 Sulfierkolben mit Rührer, Kühler, Tropftrichter und Innenthermometer werden 61,4 g (0,5 Mol) 4-Hydroxybenzonitril in 500 ml Methylcellosolve vorgelegt. Nach Erwärmen des Ansatzes auf 80°C lässt man unter kräftigem Rühren langsam 73,3 g 30%-ige NaOH (0,55 Mol) einlaufen. Es wird 15 Minuten nachgerührt, bevor man während 30 Minuten 116,8 g (0,575 Mol) 3-Brommethylheptan zutropft. Die Reaktion wird für ca. 12 Stunden bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 500 ml Toluol auf. Es wird dreimal mit Wasser ausgeschüttelt, über Natriumsulfat getrocknet und zur Trockne eingedampft. Nach Hochvakuumdestillation über eine 10cm Vigreux-Kolonne (127-132°C, 0,15 mm) erhält man 97,5 g (84% d. Th.) 4-(2-Ethylhexyloxy)-benzonitril als farbiges Öl.

b) In einm 1,51 Planschliffreaktor, ausgestattet mit Rührer, Kühler, Innenthermometer und Gaseinleitungsrohr, werden 208,7 g (0,9 Mol) 4-(2-Ethylhexyloxybenzonitril) sowie 39,8 g (1,22 Mol) Methanol in 400 ml Dichlorethan vorgelegt. Innerhalb von 5 Stunden leitet man unter starkem Rühren und Eiskühlung (0-1°C) 85,4 g (2,37 Mol) Chlorwasserstoffgas ein. Nach 24-stündigem Rühren bei Raumtemperatur zeigt das Dünnschichtchromatogramm eine quantitative Umsetzung zum Imidoeseter. Man zieht das Lösungsmittel im Vakuum ab und lässt den gelben, viskosen Rückstand unter Eiskühlung (0-10°C) innerhalb von 30 Minuten in eine gut gerührte Lösung von 34 g (2,0 Mol) Ammoniak in 800 ml Methanol einlaufen. Man rührt 1 Stunde bei Raumtemperatur, dann weitere 90 Minuten bei 50-60°C nach. Der Ansatz wird im Vakuum zur Trockne eingedampft, der schmierige Rückstand dann in 800 ml warmem Toluol/Ethanol (8/2) verrührt und über Kieselgel filtriert. Dadurch wird ein Grossteil des anfallenden Ammoniumchlorids abgetrennt. Nach Einengen des Filtrats wird diese Reinigung noch zweimal wiederholt. Man erhält 205 g (80% d.Th.) Amidiniumsalz (Fp. 172-173°C), welches noch geringe Mengen Ammoniumchlorid enthält.

c) In einem 2,51 Sulfierkolben mit Rüher, Kühler, Innenthermometer und Tropftrichter und pH-Elektrode werden 113,9 g (0,4 Mol) des in b) erhaltenen Amidiniumsalzes in einem Gemisch von 1000 ml Wasser (dest.) und 100 ml Aceton suspendiert. Bei 15-20°C werden 106,7 g 30%-ige Natronlauge (0,8 Mol) während 30 Minuten langsam zugegeben. Anschliessend tropft man 45,6 g (0,42 Mol) Chlorameisensäureethylester innerhalb einer Stunde zu (Innentemperatur: 15-20°C). Im Laufe der Umsetzung sinkt der pH-Wert von 13 (Anfangswert) auf 7,0-7,5 ab und man erhält eine körnige Suspension. Nach Zugabe von 500 ml 1,2-Dichlorbenzol wird unter Rühren auf 80°C erwärmt. Man trennt die organische Phase im Scheidetrichter ab, überführt sie in einen 1,51 Sulfierkolben (ausgestattet mit einem Liebigkühler) und erhitzt unter leichtem Vakuum (ca. 800 mbar) auf 145-175°C (Innentemperatur). Das bei der Ringschlusskondensation entstehende Urethan wird abdestilliert (Dauer ca. 90 Minuten). Man lässt die braune Reaktionsmasse bei 60°C in 600 ml Isopropanol einlaufen, saugt den Niederschlag in der Kälte (5°C) ab und wäscht mit Isopropanol, Wasser und Methanol. Anschliessend wird im Vakuum getrocknet (100°C). Das Produkt 4,6-Bis[4-(2-ethyl-hexyloxy)-phenyl]-s-triazin-2-ol zeigt eine blaue Fluoreszenz und ist dünnschichtchromatographisch einheitlich. Die Ausbeute beträgt 57 g (56 % d. Th.; Fp. 168-170°C).

d) In einem 1,51 Sulfierkolben mit Rührer, Kühler, Innenthermometer, Tropftrichter und Gasableitung werden 55,6 g (0,11 Mol)
4,6-Bis[4-(2-ethyl-hexyloxy)-phenyl]-s-triazin-2-ol in 300 ml Xylol, versetzt mit 1 ml Dimethylformamid, vorgelegt. Bei 75-80°C Innentemperatur tropft man unter starkem Rühren während 15 Minuten 17,0 g (0,14 Mol) Thionylchlorid zu. Nach Abklingen der Gasentwicklung wird die Temperatur auf 100°C erhöht. Nach 2 Stunden ist die Umsetzung beendet (Prüfung durch Dünnschichtchromatographie). Überschüssiges Thionylchorid wird unter leichtem Vakuum aus dem Reaktionsgefäss abdestilliert, das Zwischenprodukt 2-Chloro-4,6-bis-[4-(2-ethyl-hexyloxy)-phenyl]-s-triazin wird direkt weiter umgesetzt. Man trägt bei 50°C 16,2 g (0,12 Mol) trockenes, sublimiertes Aluminiumchlorid ein (ca. 1 Minute), wodurch die Temperatur auf 65°C ansteigt. Die anfangs klare, gelbe Lösung wird zu einer roten, dann olivefarbenen Suspension. Bei 50-55°C werden portionsweise 13,3 g (0,12 Mol) Resorcin zugegeben (ca. 10 Minuten), anschliessend wird auf 85°C erwärmt. Nach 3 Stunden ist das Dünnschichtchromatogramm frei von Edukt. Man kühlt auf 70°C ab und hydrolisiert den Aluminiumkomplex durch langsames Zutropfen von 300 ml 5%-iger Salzsäure, wobei die Temperatur von 80°C nicht überschritten werden sollte. Das Lösungsmittel (Xylol) wird durch Wasserdampfdestillation entfernt, der schmierige Rückstand in 500 ml heissem Toluol verrührt und über Kieselgel filtriert. Das Filtrat wird mit Aktivkohle digeriert und nochmals filtriert. Man trocknet mit Natriumsulfat und destilliert das Lösungsmittel ab. Der braune, viskose Rückstand (69 g) wird zur weiteren Reinigung in 150 ml Toluol/Essigester (95/5) gelöst und einer Säulenchromatographie unterworfen (6 cm X 60 cm Kieselgel 60). Man erhält 33,6 g (51% d.Th.) 4- {4,6-Bis-[4-(2-ethyl-hexloxy)-phenyl]-s-triazin-2-yl }-1,3-dihydroxybenzol als gelbes viskoses Öl.

e) 83,7 g (0,14 Mol) 4-{4,6-Bis-[4-(2-ethyl-hexloxy)-phenyl]-s-triazin-2-yl}-1,3- dihydroxybenzol werden zusammen mit 500 ml Methylcellosolve in einem 11 Sulfierkolben mit Rührer, Kühler, Innenthermometer und Tropftrichter bei 80°C vorgelegt. Man gibt 18,1 g einer 30%-igen Natronlauge (0,16 Mol) zu, lässt 15 Minuten nachrühren und tropft dann während 30 Minuten eine Lösung von 31,4 g (0,16 Mol) 3-Brommethylheptan und 30 ml Methylcellosolve zu. Nach 24 Stunden Rühren bei 110°C ist die Alkylierung beendet (Dünnschichtchromatogramm). Es wird unter Vakuum zur Trockne eingedampft, der Rückstand in 500 ml Toluol gelöst, filtriert und mit Wasser ausgeschüttelt. Nach Trocknung über Natriumsulfat und Abdestillieren des Lösungsmittels erhält man 100,9 g eines rotbraunen Öls. Das Rohprodukt wird in 200 ml Toluol/Essigester (97,5/2,5) gelöst und zur Reinigung über Kieselgel (10 cmx40 cm) chromatographiert. Man erhält 79,4 g (80% d.Th.) der Verbindung der Formel 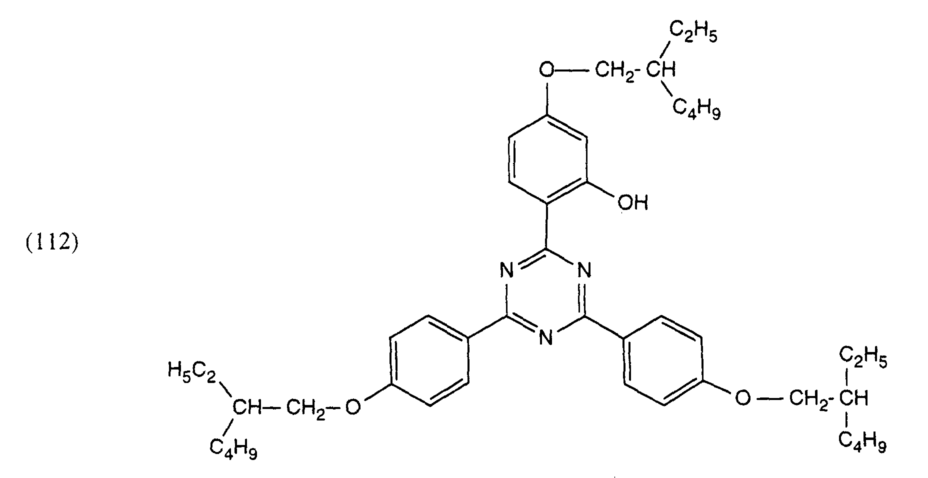 als honigartiges, rotbraunes Harz.

### Beispiel 13:4-{4,6-Bis-[4-(2-ethylhexyloxy)-phenyl]-s-triazin-2-yl}-3-hydroxy-phenoxy)-essigsäureethylester

a) In einem 1,51 Sulfierkolben mit Rührer, Kühler, Tropftrichter und Gasableitung werden 154,1 g (1,0 Mol) 2,4-Dihydroxybenzoesäure, 141,2 g (1,5 Mol) Phenol, 500 ml Toluol sowie 1 ml Dimethylformamid vorgelegt. Bei 100-105°C Innentemperatur werdenn innerhalb von 2,5 Stunden 178,5 g (1,5 Mol) Thionylchlorid zugetropft. Anschliessend wird die rötliche, klare Lösung über Nacht unter Rückfluss gehalten (110-115°C). Man destilliert Toluol und Phenol im Vakuum ab, verrührt den hochviskosen Rückstand (271,5 g) in 300 ml Toluol/Cyclohexan (7/3) und lässt über Nacht auskristallisieren. Der Niederschlag wird kalt (10°C) abgesaugt und mit 3x50 ml Toluol/Cyclohexan (7/3) gewaschen. Es wird bei 80°C im Vakuum getrocknet. Man erhält 125,5 g (54,4% d. Th.) 2,4-Dihydroxybenzoesäurephenylester (Fp. 135-137°C).

b) In einem 750 ml Sulfierkolben mit Rührer, Kühler, Innenthermometer und Tropftrichter werden 200 ml Ethanol abs. und 12,1 g (0,22 Mol) Natriumethanolat bei Raumtemperatur vorgelegt. Man trägt 59,9 g (0,21 Mol) Amidiniumhydrochlorid (Herstellung vgl. Beispiel 12 b)) ein und rührt 30 Minuten nach. Anschliessend wird das ausgefallene Kochsalz abfiltriert (Kieselgel). Eine Lösung von 23,0 g (0,1 Mol) 2,4-Dihydroxybenzoesäurephenylester (hergestellt in Beispiel 13 a)) in 100 ml Ethanol abs. wird bei Raumtemperatur zugegeben, die klare rotgelbe Lösung wird 3 Stunden unter Rückfluss gerührt (78°C). Man destilliert ca. 150 ml Ethanol ab und fügt dasselbe Volumen Ethylcellosolve hinzu. Der Ansatz wird über Nacht unter Rückfluss gehalten (90°C). Man dampft im Vakuum zur Trockne ein, verrührt zweimal mit heissem Wasser und löst in 500 ml Toluol. Nach Filtration der trüben Lösung über Kieselgel wird mit Natriumsulfat getocknet und das Lösungsmittel im Vakuum abgezogen. Das Rohprodukt (68,2 g) wird in 120 ml Toluol gelöst und über Kieselgel 60 (6 cm x 60 cm) chromatographiert (Laufmittel Toluol/Essigester 95/5). Man isoliert 28,5 g (48% d.Th.) 4-{4,6-Bis-[4-(2-ethylhexyloxy)-phenyl]-s-triazin-2-yl}-1,3-dihydroxybenzol als gelbes, viskoses Öl.

c) 14,4 g (0,024 Mol) der in b) hergestellten Verbindung werden zusammen mit 150 ml Ethanol abs. in einem 250 ml Sulfierkolben mit Rüher, Kühler, Innenthermometer und Tropftrichter bei 60°C vorgelegt. Man gibt 3,4 g einer 30%-igen Natronlauge (0,025 Mol) zu, lässt 15 Minuten nachrühren und tropft dann während 10 Minuten 4,8 g (0,028 Mol) Bromessigsäureethylester zu. Nach 24 Stunden Rühren unter Rückfluss ist die Alkylierung beendet (Dünnschichtchromatogramm). Es wird unter Vakuum zur Trockne eingedampft, der Rückstand in 250 ml Toluol aufgenommen und zweimal mit Wasser ausgeschüttelt. Nach Trocknung über Natriumsulfat und Abdestilllieren des Lösungsmittels erhält man 17,1 g eines rotbraunen Öls. Das Rohprodukt wird in 50 ml Toluol gelöst und zur Reinigung über Kieselgel chromatographiert. Man erhält 9,0 g (55% d.Th.) der Verbindung der Formel 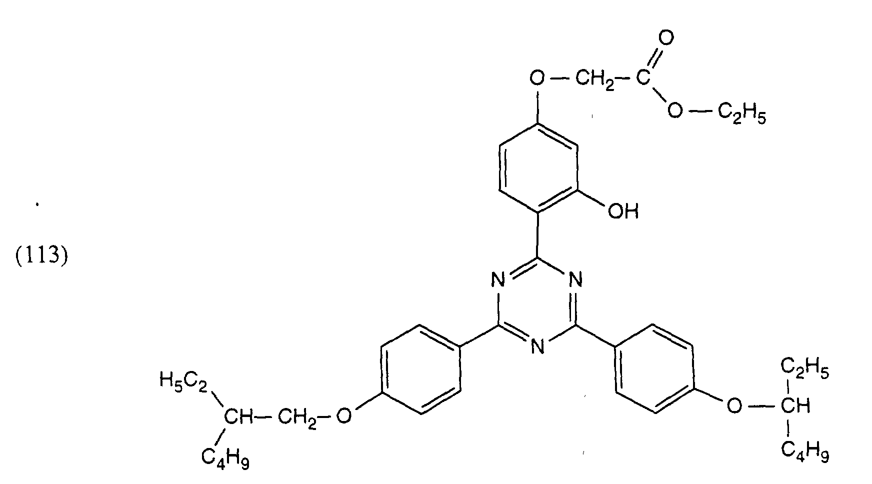 als oranges Harz, welches nach einigen Tagen kristallisiert. (Fp. 93-95°C).

### Beispiel 14: 2-{4,6-Bis-[4-(2-ethylhexyloxy)-phenyl]-s-triazin-2-yl)-5-(2-ethylhexyl oxy)-phenol

a) In einem 11 Sulfierkolben mit Rührer, Kühler, Tropftrichter und Innenthermometer werden 86,5 g (0,5 Mol) 4-Bromphenol in 500 ml Methylcellosolve vorgelegt. Nach Erwärmen auf 60°C lässt man langsam 70,0 g 30%-ige NaOH (0,53 Mol) einlaufen. Es wird 15 Minuten nachgerührt, bevor man während 45 Minuten 116,8 g (0,575 Mol) 3-Brommethylheptan zutropft. Die Reaktion wird über Nacht bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 600 ml Toluol auf. Es wird dreimal mit Wasser ausgeschüttelt, über Natriumsulfat getrockent und zur Trockne eingedampft. Man erhält 119,3 g (84% d.Th.) 4-(2-Ethylhexyloxy)-brombenzol als leichtes gelbes Öl.

b) In einm 100 ml Sulfierkolben, ausgerüstet mit Rührer, Kühler, Trockenrohr, Tropftrichter und Innenthermometer werden unter Schutzgas (trockener Stickstoff) 3,65 g (0,15 Mol) Magnesiumspäne vorgelegt und mit einigen Kristallen Jod angeätzt. Man fügt 150 ml wasserfreies Tetrahydrofuran hinzu und tropft innerhalb von 45 Minuten eine Lösung von 42,8 g (0,15 Mol) 4-(2-Ethylhexyloxy)-brombenzol in 30 ml Tetrahydrofuran zu (Raumtemperatur). Nach leichtem Erwärmen auf dem Wasserbad (40°C) springt die Grignard-Reaktion an Trübung, Exothermie). Man rührt eine Stunde bei 40°C, dann unter Rückfluss (66°C), bis das Magnesium nahezu vollständig gelöst ist (ca. 30 Minuten). Nach Abkühlung auf Raumtemperatur wird die Grignard-Lösung bei 0-20°C innerhalb von 60 Minuten zu einer Lösung von 9,2 g (0,05 Mol) Cyanurchlorid in 40 ml Tetrahydrofuran zugetropft (350 ml Sulfierkolben, Rührer, Kühler, Trockenrohr, Tropftrichter, Innenthermometer, Schutzgas). Man rührt über Nacht unter Rückfluss (66°C) und dampft anschliessend zur Trockne ein. Der Rückstand wird in 100 ml eiskalter 2N Salzsäure verrührt und dann mit 200 ml Toluol extrahiert. Die organische Phase wird zweimal mit 10%-iger Sole geschüttelt, über Natriumsulfat getrocknet und eingedampft. Das Rohprodukt (40,3 g, rotes Öl) ist noch stark verunreinigt (Dünnschichtchromatogramm). Zur Reinigung wird in 80 ml Toluol/Hexan (1/1) gelöst und über Kieselgel (5 cm x 45 cm) chromatographiert. Man isoliert 18,2 g (69,5%) 2-Chloro-4,6-bis-[4-(2-ethyl-hexyloxy)-phenyl]-s-triazin als gelbes Harz.

c) Die Friedel-Crafts-Acylierung erfolgt wie in Beispiel 12 d) beschrieben. Man erhält das Produkt 4-{4,6-BIs-[4-(2-ethylhexyloxy)-phenyl]-s-triazin-2-yl}-1,3-diydroxybenzol.

d) Die Alkylierung erfolgt wie in Beispiel 12 e) beschrieben.

Das Endprodukt ist die Verbindung der Formel (112).

Beispiel 15 bis 17: Nach den in den Beispielen 12 bis 14 beschriebenen Verfahren sind auch die folgenden Verbindungen erhältlich (Tabelle 2):

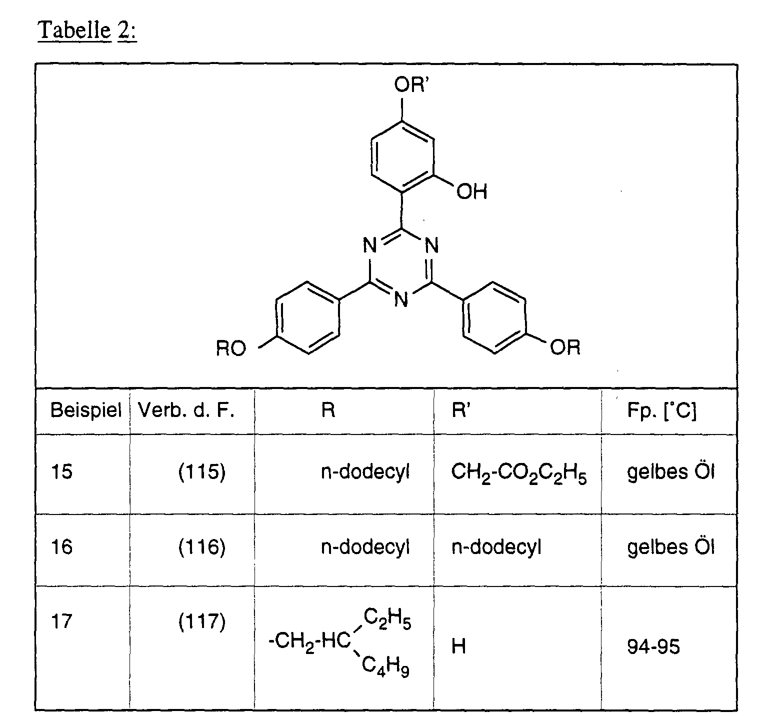

### Applikationsbeispiele

### Beispiel 23: Anwendung in organischen Polymeren

In 50 g Methylenchlorid werden 10 g Polycarbonat-Pulver (Lexan® 115) unter Rühren bei Raumtemperatur gelöst, was mehrere Stunden beansprucht. Dazu kommen 0,2 g UV-Absorber, entsprechend 2 % Zusatzkonzentration. Aus diesen Lösungen werden Filme von 20 µm Dicke gegossen.

Die Filme werden in einem Atlas Weatherometer CI 65 bei einer Schwarztafeltemperatur von 63°C, einer Strahlungsenergie von 0,35 W/m² bei 340 nm und einer relativen Feuchte von 60 % belichtet. In regelmäßigen Intervallen wird die Verfärbung der Proben durch Messung des Yellowness Index (YI, Methode ASTM D 1925) überprüft. In Tabelle 7 ist die Belichtungszeit bis zu einem Yellowness-Index von 7 angegeben.

Anschließend werden die Filme weiter belichtet bis zur Versprödung, die sich durch Bildung von Rissen in den Filmen anzeigt. Die Belichtungsdauer bis zur Versprödung ist ebenfalls in Tabelle 6 aufgeführt.

**Tabelle 6:**

| Belichtungszeit (h) bis zum Erreichen eines Yellowness Index (YI) = 7 sowie bis zum Verspröden | | |
|---|---|---|
| UV-Absorber der Formel | Belichtungszeit (h) YI=7 | bis zur Versprödung |
| keiner | 590 | 1375 |
| (111) | 2100 | 5000 |
| (101) | 1480 | 4980 |

## Patentansprüche

1. Stabilisiertes organisches Polymer, enthaltend als Stabilisator gegen Licht, Sauerstoff und Wärme 0,01 bis 5 Gew.-%, bezogen auf das organische Polymer, mindestens eines Hydroxyphenyl-s-triazins der Formel (2) 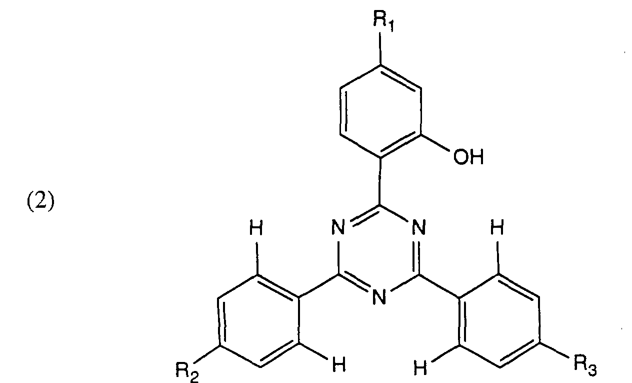 worin
R₁ Hydroxy, C₁-C₁₅-Alkyl, C₁-C₁₅-Alkoxy oder einen Rest der Formel 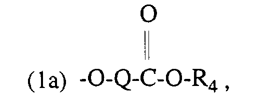
R₂ und R₃, unabhängig voneinander, Wasserstoff oder C₁-C₁₅-Alkoxy,
R₄ C₁-C₅-Alkyl oder C₁-C₅-Alkoxy-C₁-C₅-Alkyl und
Q einen C₁-C₄-Alkylenrest bedeuten, und
die Verbindung mindestens zwei C₁-C₁₅-Alkoxyreste aufweisen muss.

2. Stabilisiertes Polymer nach Anspruch 1, dadurch gekennzeichnet, dass in Formel (2)
R₁ Hydroxy, C₁-C₁₅-Alkyl, C₁-C₁₅-Alkoxy und
R₂ und R₃ unabhängig voneinander Wasserstoff oder C₁-C₁₅-Alkoxy bedeuten.

3. Stabilisiertes Polymer nach einem der Ansprüche 1 oder 2, dadurch gekennzeichnet, dass in Formel (2)
R₂ und R₃ unabhängig voneinander C₁-C₅-Alkoxy bedeuten.

4. Stabilisiertes Polymer nach einem der Ansprüche 1 oder 2, dadurch gekennzeichnet dass in Formel (2)
R₂ Wasserstoff bedeutet.

5. Stabilisiertes Polymer nach Anspruch 1, dadurch gekennzeichnet, dass in Formel (2) R₂ und R₃ Methoxy oder Ethoxy bedeuten.

6. Stabilisiertes Polymer nach Anspruch 1, dadurch gekennzeichnet, dass in Formel (2)
R₂ Wasserstoff und
R₃ Methoxy oder Ethoxy bedeuten.

7. Stabilisiertes Polymer nach Anspruch 1, dadurch gekennzeichnet, dass in Formel (2)
R₁ und R₃ Methoxy und
R₂ Wasserstoff bedeuten.

8. Stabilisiertes Polymer nach Anspruch 1, dadurch gekennzeichnet, dass in Formel (2) R₁, R₂ und R₃, unabhängig voneinander, C₅-C₁₅-Alkoxy bedeuten.

9. Stabilisiertes Polymer nach Anspruch 1, dadurch gekennzeichnet, dass in Formel (2)
R₁ einen Rest der Formel (1a) und
R₂ und R₃ C₅-C₁₅-Alkoxy bedeuten.

10. Stabilisiertes Polymer nach Anspruch 1, worin die Verbindung der Formel (2) ausgewählt ist aus
2-(2-Hydroxy-4-methoxyphenyl)-4-(4-methoxyphenyl)-6-phenyl-1,3,5-triazin;
(4-{4,6-Bis-[4-(2-ethylhexyloxy)-phenyl]-s-triazin-2-yl}-3-hydroxy-phenoxy)-essigsäureethylester;
(4- {4,6-Bis-[4-(dodecyloxy)-phenyl]-s-triazin-2-yl}-3-hydroxy-phenoxy)-essigsäureethylester.

11. Verwendung der Verbindung der Formel (2) gemäß Anspruch 1 als Stabilisator für organische Polymere gegen Schädigung durch Licht, Sauerstoff und Wärme in einer Menge von 0,01 bis 5 Gew.-%, bezogen auf das organische Polymer.

12. Verwendung nach Anspruch 11 in Lacken.

13. Stabilisiertes Polymer nach Anspruch 1, dadurch gekennzeichnet, dass das Polymer ein Bindemittel für einen Lack ist.

14. Stabilisiertes Polymer nach Anspruch 13, dadurch gekennzeichnet, dass das Polymer außerdem einen oder mehrere andere Stabilisatoren und/oder sonstige Zusatzstoffe enthält.

15. Stabilisiertes Polymer nach Anspruch 14, dadurch gekennzeichnet, dass das Polymer ein Lichtschutzmittel aus der Klasse der sterisch gehinderten Amine und/oder der Klasse der 2-(2'-Hydroxyphenyl)-benztriazole enthält.

## Claims

1. A stabilized organic polymer comprising as light, oxygen and heat stabilizer from 0.01 to 5% by weight, based on the organic polymer, of at least one hydroxyphenyl-s-triazine of the formula (2) 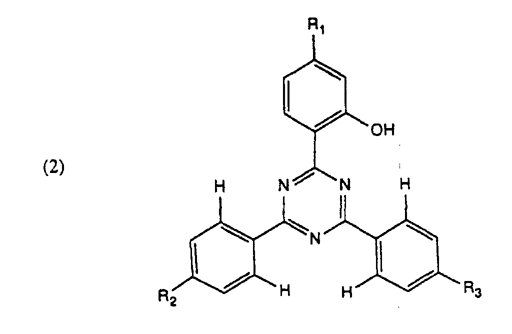 in which
R₁ is hydroxyl, C₁-C₁₅alkyl, C₁-C₁₅alkoxy or a radical of the formula 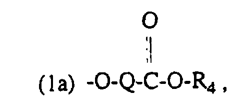
R₂ and R₃ are each independently of the other hydrogen or C₁-C₁₅alkoxy, R₄ is C₁-C₅alkyl or C₁-C₅alkoxy-C₁-C₅alkyl
and Q is a C₁-C₄alkylene radical, and the compound must contain at least two C₁-C₁₅alkoxy radicals.

2. A stabilized polymer according to claim 1, wherein in formula (2) R₁ is hydroxyl, C₁-C₁₅alkyl or C₁-C₁₅alkoxy and R₂ and R₃ are each independently of the other hydrogen or C₁-C₁₅alkoxy.

3. A stabilized polymer according to claim 1 or 2, wherein in formula (2) R₂ and R₃ are each independently of the other C₁-C₅alkoxy.

4. A stabilized polymer according to claim 1 or 2, wherein in formula (2) R₂ is hydrogen.

5. A stabilized polymer according to claim 1, wherein in formula (2) R₂ and R₃ are methoxy or ethoxy.

6. A stabilized polymer according to claim 1, wherein in formula (2) R₂ is hydrogen and R₃ is methoxy or ethoxy.

7. A stabilized polymer according to claim 1, wherein in formula (2) R₁ and R₃ are methoxy and R₂ is hydrogen.

8. A stabilized polymer according to claim 1, wherein in formula (2) R₁, R₂ and R₃ are each independently of one another C₅-C₁₅alkoxy.

9. A stabilized polymer according to claim 1, wherein in formula (2) R₁ is a radical of the formula (1a) and R₂ and R₃ are C₅-C₁₅alkoxy.

10. A stabilized polymer according to claim 1, in which the compound of the formula (2) is selected from
2-(2-hydroxy-4-methoxyphenyl)-4-(4-methoxyphenyl)-6-phenyl-1,3,5-triazine;
ethyl (4-{4,6-bis[4-(2-ethylhexyloxy)phenyl]-s-triazin-2-yl}-3-hydroxyphenoxy)acetate;
ethyl (4-{4,6-bis[4-(dodecyloxy)phenyl]-s-triazin-2-yl}-3-hydroxyphenoxy)acetate.

11. The use of the compound of the formula (2) of claim 1 as a stabilizer for organic polymers against damage by light, oxygen and heat in an amount of from 0.01 to 5% by weight, based on the organic polymer.

12. The use according to claim 11 in coating materials.

13. A stabilized polymer according to claim 1, which is a binder for a coating material.

14. A stabilized polymer according to claim 13, further comprising one or more different stabilizers and/or other additives.

15. A stabilized polymer according to claim 14, comprising a light stabilizer from the class of the sterically hindered amines and/or of the 2-(2'-hydroxyphenyl)benzotriazoles.

## Revendications

1. Polymère organique stabilisé contenant, en tant que stabilisant contre la lumière, l'oxygène et la chaleur, de 0,01 à 5 % en masse, par rapport au polymère organique, au moins une hydroxyphényl-s-triazine de formule (2) 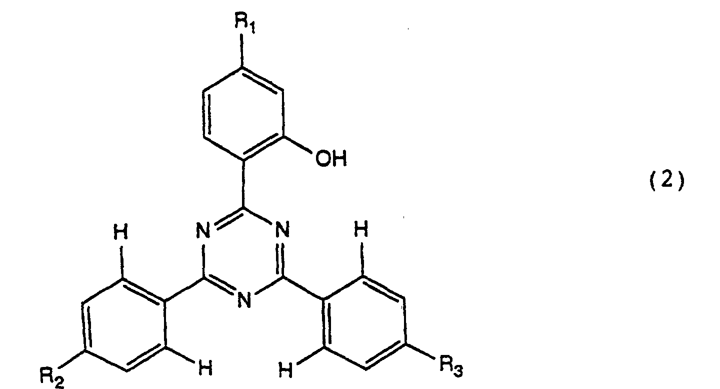 où
R₁ représente des groupes hydroxy, alkyle en C₁-C₁₅, alkoxy en C₁-C₁₅ ou un reste de formule 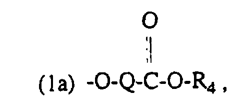
R₂ et R₃ représentent, indépendamment l'un de l'autre, un atome d'hydrogène ou un groupe alkoxy en C₁-C₁₅,
R₄ représente un groupe alkyle en C₁-C₁₅ ou (alkoxy en C₁-C₁₅)-alkyle en C₁-C₁₅ et
Q représente un reste alkylène en C₁-C₄, et
le composé doit présenter au moins deux restes alkoxy en C₁-C₁₅.

2. Polymère stabilisé selon la revendication 1, caractérisé en ce que dans la formule (2)
R₁ représente des groupes hydroxy, alkyle en C₁-C₁₅, alkoxy en C₁-C₁₅ et
R₂ et R₃ représentent, indépendamment l'un de l'autre, un atome d'hydrogène ou un groupe alkoxy en C₁-C₁₅.

3. Polymère stabilisé selon l'une des revendications 1 ou 2, caractérisé en ce que dans la formule (2)
R₂ et R₃ représentent, indépendamment l'un de l'autre, un groupe alkoxy en C₁-C₁₅.

4. Polymère stabilisé selon l'une des revendications 1 ou 2, caractérisé en ce que dans la formule (2)
R₂ représente un atome d'hydrogène.

5. Polymère stabilisé selon la revendication 1, caractérisé en ce que dans la formule (2)
R₂ et R₃ représentent des groupes méthoxy ou éthoxy.

6. Polymère stabilisé selon la revendication 1, caractérisé en ce que dans la formule (2)
R₂ représente un atome d'hydrogène et
R₃ représente des groupes méthoxy ou éthoxy.

7. Polymère stabilisé selon la revendication 1, caractérisé en ce que dans la formule (2)
R₁ et R₃ représentent un groupe méthoxy et
R₂ représente un atome d'hydrogène.

8. Polymère stabilisé selon la revendication 1, caractérisé en ce que dans la formule (2)
R₁, R₂ et R₃ représentent, indépendamment les uns des autres, un groupe alkoxy en C₅-C₁₅.

9. Polymère stabilisé selon la revendication 1, caractérisé en ce que dans la formule (2)
R₁ représentent un reste de formule (1a) et
R₂ et R₃ représentent un groupe alkoxy en C₅-C₁₅·

10. Polymère stabilisé selon la revendication 1, où le composé de formule (2) est pris parmi les
2-(2-hydroxy-4-méthoxyphényl)-4-(4-méthoxyphényl)-6-phényl-1,3,5-triazine ;
(4-{4,6-bis-[4-(2-éthylhexyloxy)-phényl]-s-triazin-2-yl}-3-hydroxy-phénoxy)acétate d'éthyle ;
(4-{4,6-bis-[4-(dodécyloxy)-phényl]-s-triazin-2-yl}-3-hydroxy-phénoxy)acétate d'éthyle.

11. Utilisation du composé de formule (2) selon la revendication 1 en tant qua stabilisant de polymères organiques contre la dégradation induite par la lumière, l'oxygène et la chaleur, en une quantité de 0,01 à 5 % en masse, par rapport au polymère organique.

12. Utilisation selon la revendication 11 dans des vernis.

13. Polymère stabilisé selon la revendication 1, caractérisé en ce que le polymère est un liant pour un vernis.

14. Polymère stabilisé selon la revendication 13, caractérisé en ce que le polymère contient de plus un ou plusieurs autres stabilisants et/ou autres matières d'addition.

15. Polymère stabilisé selon la revendication 14, caractérisé en ce que le polymère contient un agent photoprotecteur de la classe des amines à encombrement stérique et/ou de la classe des 2-(2'-hydroxyphényl)benzotriazoles.
